# FASCICULE DE BREVET EUROPEEN

(11) **EP 3 046 488 B1**
(45) Date de publication et mention de la délivrance du brevet: **13.06.2018**
(21) Numéro de dépôt: 14705378.9
(22) Date de dépôt: 21.01.2014
(51) Int. Cl.: A61B 17/64

(54) **DISPOSITIF DE FIXATION EXTERNE POUR OSTEOSYNTHESE DE L'EPAULE**
EXTERNER FIXATEUR ZUR OSTEOSYNTHESE DER SCHULTER
EXTERNAL FIXATOR FOR OSTEOSYNTHESIS OF THE SHOULDER

(30) Priorité: 19.09.2013 FR 1359003
(43) Date de publication de la demande: 27.07.2016
(73) Titulaire: Assut Europe S.p.A., 00173 Rome (IT)
(72) Inventeur: PERRET, Jean-Pierre, F-74300 Thyez (FR)
(74) Mandataire: Gasquet, Denis
(86) Numéro de dépôt international: PCT/FR2014/050106
(87) Numéro de publication internationale: WO 2015/040291

(56) Documents cités:
- WO-A1-2012/072756
- DE-A1- 3 244 819
- US-A- 2 391 537
- US-A1- 2003 216 734
- US-A1- 2009 024 128
- US-A1- 2009 228 006

## Description

La présente invention concerne un dispositif pour maintenir en place des parties d'os. Elle concerne plus particulièrement un dispositif externe de maintien destiné à la réparation et à la réduction d'une fracture de la tête de l'humérus d'un patient.

Les dispositifs externes destinés à maintenir des parties d'os sont connus sous l'expression "fixateurs externes". Ils sont destinés à immobiliser des parties d'os grâce à un support se trouvant à l'extérieur et condamnant tout mouvement relatif, assurant ainsi la soudure des parties après fracture. On connait par exemple les dispositifs décrits dans les demandes de brevet français N° 89 12388, 85 02577 et 83 19288. Mais ces dispositifs connus ne permettent pas la réparation des fractures de la tête de l'humérus d'un patient par voie externe. La demande de brevet international WO 2012/072756 A1 divulgue un dispositif externe selon le préambule de la revendication 1. Le but de la présente invention est de proposer un dispositif externe destiné à la réparation et à la réduction d'une fracture de la tête de l'humérus.

Ainsi la présente invention fournit un dispositif externe de maintien destiné à la réparation et à la réduction d'une fracture de la tête de l'humérus d'un patient selon la revendication 1. Selon une caractéristique complémentaire, la tige support comprend deux ensembles de retenue, ces derniers étant chacun destinés à retenir une broche transversale respectivement destinée à être impactée ou vissée par leur extrémité dans le corps de l'humérus afin de retenir la tige support à pour en assurer sa fixation sans mouvement possible.

Selon une autre caractéristique, chacun des ensembles de retenue est constitué par un organe principal et un organe secondaire, tandis que l'organe principal est une pièce volumique ayant avantageusement la forme chape fendue comprenant un trou pour le passage de la tige support tandis qu'une vis assure le serrage de la chape sur la tige support.

Selon une autre caractéristique complémentaire, à chaque organe principal est associé l'organe secondaire, ce dernier étant constitué comme l'organe principal par une chape fendue fixée par serrage sur l'organe principal grâce à la vis, tandis que l'organe secondaire comprend un trou central dans lequel est engagée la broche transversale.

Ajoutons que les broches de retenue sont fixées à la tige support en partie haute.

Notons aussi que l'extrémité supérieure de la tige support comprend un ensemble supérieur de retenue.

Précisons que l'ensemble supérieur de retenue est constitué par une tige transversale retenue en partie haute de la tige support grâce à une douille percée d'au moins un trou transversal dans lequel est retenue la tige transversale avantageusement dans une position inclinée pour s'étendre vers l'extérieur et vers le haut, la dite branche transversale étant retenue dans le trou grâce à une vis de fixation.

Précisons aussi, que chacune des broches de retenue est fixée sur la tige transversale par un ensemble de retenue (11a, 11b, 11c) identique aux ensembles de retenue permettant la fixation des broches transversales.

Selon l'invention, le dispositif comprend trois broches de retenue, tandis que l'une des broches est fixée directement sur la tige transversale, alors que les deux autres sont fixées sur une deuxième tige transversale fixée sur la tige transversale.

La tige support, selon le mode de réalisation préféré, est constituée de deux éléments de tige, à savoir un élément de tige inférieur et un élément de tige supérieur afin de former un ensemble télescopique, tandis qu'elle comprend des moyens de réglage en longueur permettant d'ajuster la longueur de la tige support par déplacement longitudinal d'un élément de tige par rapport à l'autre élément.

D'autres caractéristiques et avantages de l'invention se dégageront de la description qui va suivre en regard des dessins annexés qui ne sont donnés qu'à titre d'exemples non limitatifs.
La figure 1 est une vue en perspective du dispositif en cours d'utilisation.

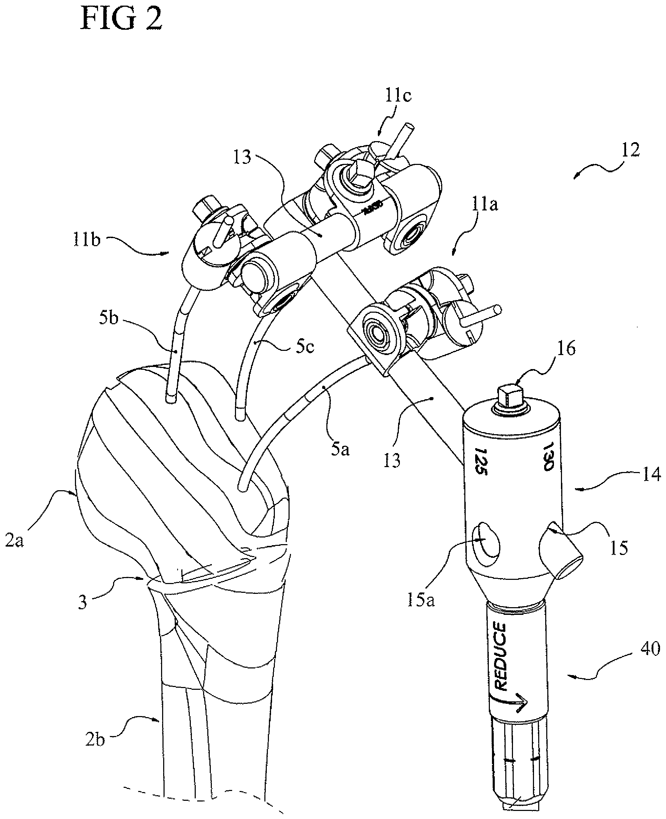

La figure 2 est une vue de détail du dispositif en cours d'utilisation.

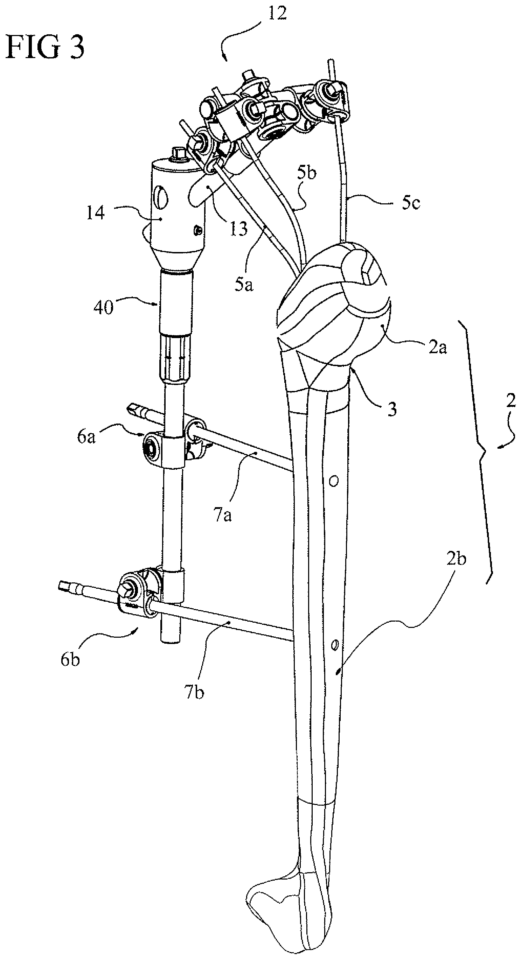

La figure 3 est une autre vue en perspective du dispositif en cours d'utilisation.

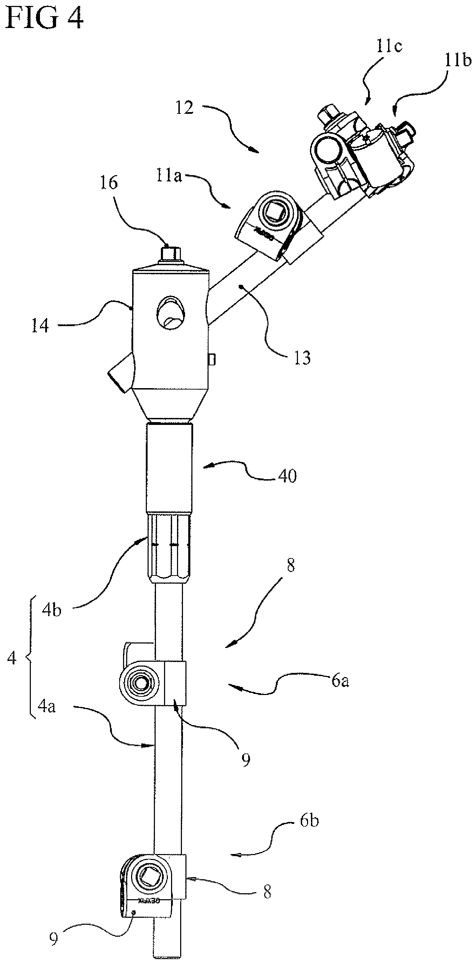

La figure 4 est une vue latérale du dispositif seul sans les différentes broches associées.

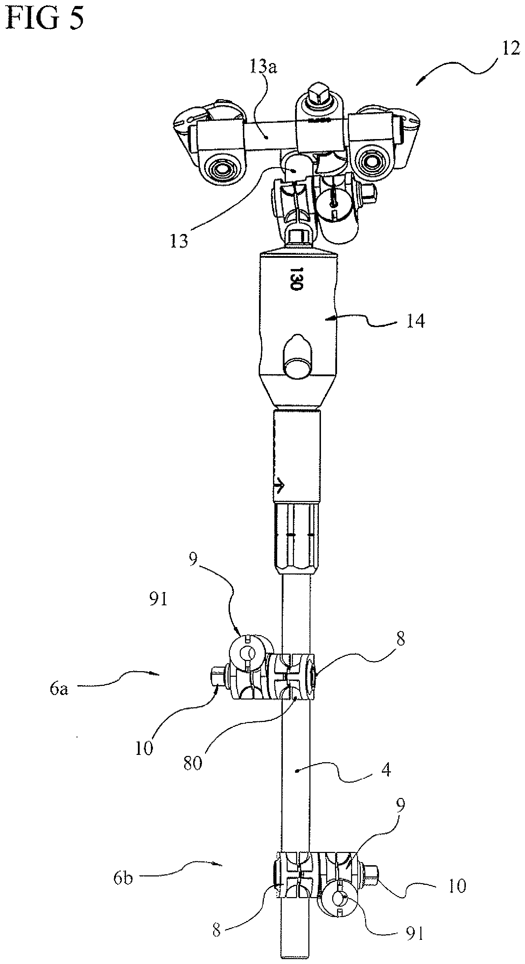

La figure 5 est une vue arrière du dispositif seul sans les différentes broches qui lui sont associées.

Le dispositif de l'invention (1) est un dispositif externe de maintien destiné à la réparation d'une rupture de la tête de l'humérus d'un patient, en maintenant en place par voie externe les deux parties (2a, 2b) de l'humérus (2), la tête de (2a) de l'humérus (2) étant fracturée (3) du corps (2b) l'humérus.

Le dispositif de l'invention (1) comprend une tige support (4) destinée à retenir au moins deux broches de retenue (5a, 5b) destinées à être insérées sensiblement verticalement aussi bien dans la tête de l'humérus que dans le corps (2b) proprement dit de l'humérus (2).

A cet effet la tige support (4) est de forme générale cylindrique et comprend avantageusement des moyens de réglage en longueur (40) permettant d'ajuster la longueur de la tige support, ayant pour effet de rapprocher les os et ainsi réduire la fracture.

Précisons que la tige support (4) comprend deux ensembles de retenue (6a, 6b). Chacun des ensembles de retenue est destiné à retenir une broche transversale respectivement (7a, 7b) destinée à être impactée ou vissée par leur extrémité dans le corps de l'humérus (2b) afin de retenir la tige support (4) pour en assurer sa fixation sans mouvement possible. Notons que la tige support (4), une fois fixée sur l'humérus, est sensiblement parallèle au corps de l'humérus.

Chacun des ensembles de retenue (6a, 6b) permet de retenir fermement la broche transversale correspondante (7a, 7b) sur la tige support (4).

Chacun des ensembles de retenue (6a, 6b) constitue un organe d'articulation qui permet de positionner correctement chaque broche transversale (7a, 7b) par rapport à la tige support (4). Grâce aux ensemble de retenue (6a, 6b) chacune des broches transversale (7a, 7b) peut être positionnée à la bonne hauteur et avec une bonne orientation.

Chacun des ensembles de retenue est constitué par un organe principal (8) et un organe secondaire (9). L'organe principal (8) est une pièce volumique ayant avantageusement la forme chape fendue comprenant un trou (80) pour le passage de la tige support (4) tandis qu'une vis (10) assure le serrage de la chape sur la tige support. A chaque organe principal (8) est associé l'organe secondaire (9). Ce dernier (9) est constitué comme l'organe principal par une chape fendue fixée par serrage sur l'organe principal grâce à la vis (10). On notera que la vis (10) sert aussi d'axe de positionnement angulaire de l'organe secondaire par rapport à l'organe principal. Par ailleurs la chape de l'organe secondaire (9) comprend un trou central (91) dans lequel est engagée la broche transversale (7a, 7b). Selon le mode de réalisation illustré, le dispositif de l'invention (1) comprend au moins deux broches de retenue (5a, 5b) destinées à être insérées sensiblement verticalement aussi bien dans la tête de l'humérus que dans le corps (2b) proprement dit de l'humérus (2). Le dispositif de l'invention comprend trois broches de retenue (5a, 5b, 5c). Ces dernières sont fixées à la tige support (4) en partie haute.

A cet effet l'extrémité supérieure de la tige support (4) comprend un ensemble supérieur de retenue (12). Ce dernier (12) est constitué par une tige transversale (13) retenue en partie haute de la tige support (4) grâce à une douille (14) percée d'au moins un trou transversal (15) dans lequel est retenu la tige transversale (13) avantageusement dans une position inclinée pour s'étendre vers l'extérieur et vers le haut. La branche transversale est retenue dans le trou grâce à une vis de fixation (16).

Chacune des broches de retenue (5a, 5b, 5c) est fixée sur la tige transversale (13) par des ensembles de retenue (11a, 11b, 11c) identiques aux ensembles de retenue (6a,6b) décrits précédemment dans le cadre de la fixation des broches transversales (7a, 7b). Selon l'invention, le dispositif comprend trois broches de retenue (5a, 5b, 5c) l'une des broches (5a) est fixée directement sur la tige transversale (13) tandis que les deux autres (5b, 5c) sont fixées sur une deuxième tige transversale (13a) fixée sur la tige transversale (13) pour s'étendre perpendiculairement à ladite tige transversale (13).

On notera que la tige support (4) est constituée de deux éléments de tige, à savoir un élément de tige inférieur (4a) et un élément de tige supérieur (4b) afin de former un ensemble télescopique. Ajoutons que grâce aux moyens de réglage en longueur (40) il est possible d'ajuster la longueur de la tige support par déplacement longitudinal d'un élément de tige par rapport à l'autre élément.

On notera que selon le mode de réalisation préféré et illustré les ensembles de retenue (6a, 6b) des broches transversales (7a, 7b) sont disposés sur l'élément inférieur de tige support (4a).

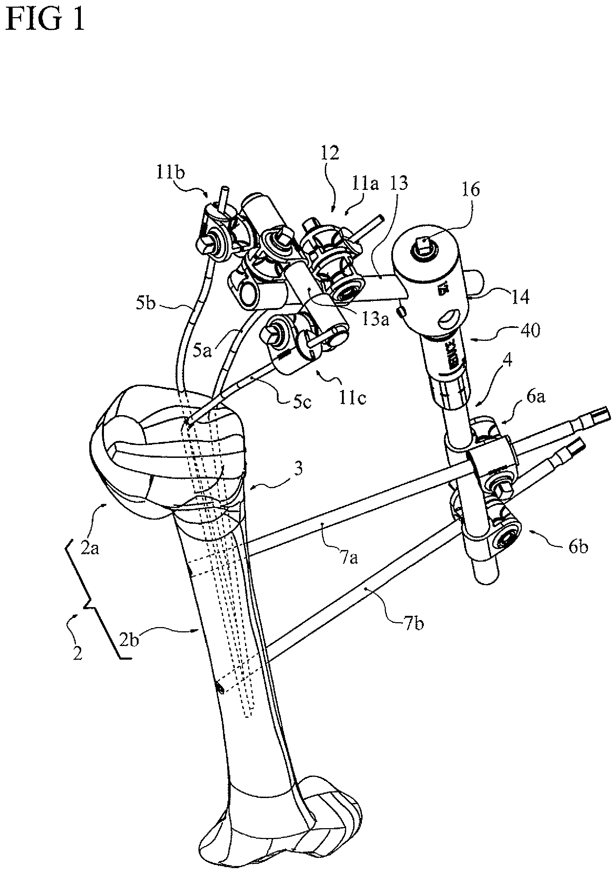

Ajoutons que les broches de retenue (5a, 5b, 5c) sont en matériau déformable pour pouvoir être courbées en sortie de leur ensemble de retenue, mais suffisamment rigide pour être introduites dans un premier temps dans la tête (2a) de l'humérus puis dans le corps (2b) de l'humérus tel que cela apparait plus particulièrement à la figure 1.

Selon le mode opératoire et tel que cela apparait sur les illustrations, les broches de retenues (5a, 5b, 5c) sont introduites dans l'humérus de façon à ce qu'elles soient convergentes vers le bas, ce qui assure le serrage et le maintien de la tête de l'humérus au corps de l'humérus, et ainsi permettre la réduction de la fracture et sa réparation. Ajoutons que grâce au moyen de réglage en longueur (40) de la tige support, le chirurgien pourra régler la force d'application de la tête (2a) de l'humérus au corps (2b) de l'humérus, afin d'accélérer la soudure des deux parties d'os.

Bien entendu, les ensembles de retenue (6a, 6b, 6d) pourraient être comme par exemple ceux décrit dans les demandes de brevet WO 88/01152, FR 92009201, UK 2 033 758.

## Revendications

1. Dispositif externe (1) de maintien destiné à la réparation et à la réduction d'une fracture de la tête de l'humérus d'un patient, en maintenant en place par voie externe les deux parties (2a, 2b) de l'humérus (2), tandis qu'il comprend une tige support (4) dont l' extrémité supérieure comprend un ensemble supérieur de retenue (12) qui est constitué par une tige transversale (13) retenue en partie haute de la tige support (4), **caractérisé en ce qu'**il comprend trois broches de retenue (5a, 5b, 5c), l'une des broches (5a) étant fixée directement sur la tige transversale, (13) tandis que les deux autres (5b, 5c) sont fixées sur une deuxième tige transversale (13a) fixée sur la tige transversale (13).

2. Dispositif (1) selon la revendication précédente, **caractérisée en ce que** la tige support (4) comprend deux ensembles de retenue (6a, 6b), ces derniers étant chacun destinés à retenir une broche transversale respectivement (7a, 7b) destinée à être impactée ou vissée par leur extrémité dans le corps de l'humérus (2b) afin de retenir la tige support (4) pour en assurer sa fixation sans mouvement possible.

3. Dispositif (1) selon la revendication 2, **caractérisée en ce que** chacun des ensembles de retenue (6a, 6b) est constitué par un organe principal (8) et un organe secondaire (9).

4. Dispositif (1) selon la revendication 3, **caractérisé en ce que** l'organe principal (8) est une pièce volumique ayant avantageusement la forme chape fendue comprenant un trou (80) pour le passage de la tige support (4) tandis qu'une vis (10) assure le serrage de la chape sur la tige support.

5. Dispositif (1) selon la revendication précédente, **caractérisé en ce qu'**à chaque organe principal (8) est associé l'organe secondaire (9), ce dernier (9) étant constitué comme l'organe principal par une chape fendue fixée par serrage sur l'organe principal grâce à la vis (10), tandis que l'organe secondaire (9) comprend un trou central (91) dans lequel est engagée la broche transversale (7a, 7b).

6. Dispositif (1) selon la revendication précédente, **caractérisé en ce que** la tige transversale (13) est retenue en partie haute de la tige support (4) grâce à une douille (14) percée d'au moins un trou transversal (15) dans lequel est retenue la tige transversale (13) avantageusement dans une position inclinée pour s'étendre vers l'extérieur et vers le haut, la dite branche transversale étant retenue dans le trou grâce à une vis de fixation (16).

7. Dispositif (1) selon la revendication précédente, **caractérisé en ce que** chacune des broches de retenue (5a, 5b, 5c) est fixée sur la tige transversale (13) par des ensembles de retenue (11a, 11b, 11c) identiques aux ensembles de retenue (6a, 6b) permettant la fixation des broches transversales (7a, 7b).

8. Dispositif (1) selon l'une quelconque des revendications précédentes, **caractérisé en ce que** la tige support (4) est constituée de deux éléments de tige, à savoir un élément de tige inférieur (4a) et un élément de tige supérieur (4b) afin de former un ensemble télescopique, tandis qu'elle comprend des moyens de réglage en longueur (40) permettant d'ajuster la longueur de la tige support par déplacement longitudinal d'un élément de tige par rapport à l'autre élément.

## Patentansprüche

1. Externer Fixateur (1), der für die Reparatur und die Reduktion eines Bruchs des Humeruskopfes eines Patienten vorgesehen ist, wobei die zwei Teile (2a, 2b) des Humerus (2) auf externem Weg an Ort und Stelle gehalten werden, während er eine Haltestange (4) umfasst, deren oberes Ende eine obere Halteeinheit (12) umfasst, die von einer Querstange (13) gebildet ist, die im oberen Teil der Haltestange (4) gehalten wird, **dadurch gekennzeichnet, dass** er drei Haltespindeln (5a, 5b, 5c) umfasst, wobei eine der Spindeln (5a) direkt auf der Querstange (13) befestigt ist, während die zwei anderen (5b, 5c) auf einer zweiten Querstange (13a) befestigt sind, die auf der Querstange (13) befestigt ist.

2. Fixateur (1) nach dem vorhergehenden Anspruch, **dadurch gekennzeichnet**, **dadurch gekennzeichnet, dass** die Haltestange (4) zwei Halteeinheiten (6a, 6b) umfasst, wobei diese letztgenannten jeweils dazu bestimmt sind, eine Querspindel (7a, 7b), die jeweils dazu bestimmt ist, mit ihrem Ende in den Körper des Humerus (2b) geschlagen oder geschraubt zu werden, um die Haltestange (4) zu halten, um ihre Befestigung ohne Bewegungsmöglichkeit zu gewährleisten.

3. Fixateur (1) nach Anspruch 2, **dadurch gekennzeichnet, dass** jede der Halteeinheiten (6a, 6b) von einem Hauptelement (8) und einem Hilfselement (9) gebildet ist.

4. Fixateur (1) nach Anspruch 3, **dadurch gekennzeichnet, dass** das Hauptelement (8) ein Volumenteil, vorteilhafterweise in Form eines gespaltenen Gabelstücks, ist, umfassend ein Loch (80) für den Durchgang der Haltestange (4), während eine Schraube (10) die Befestigung des Gabelstücks auf der Haltestange gewährleistet.

5. Fixateur (1) nach dem vorhergehenden Anspruch, **dadurch gekennzeichnet, dass** jedem Hauptelement (8) das Hilfselement (9) zugeordnet ist, wobei dieses letztgenannte (9) wie das Hauptelement von einem gespaltenen Gabelstück gebildet ist, das durch Festklemmen auf dem Hauptelement mit Hilfe der Schraube (10) befestigt ist, während das Hilfselement (9) ein zentrales Loch (91) umfasst, in das die Querspindel (7a, 7b) eingesetzt ist.

6. Fixateur (1) nach dem vorhergehenden Anspruch, **dadurch gekennzeichnet, dass** die Querstange (13) im oberen Teil der Haltestange (4) durch eine Muffe (14) gehalten wird, die mit mindestens einem Querloch (15) durchbohrt ist, in dem die Querstange (13) vorteilhafterweise in einer geneigten Position gehalten wird, um sich nach außen und nach oben zu erweitern, wobei der Querschenkel in dem Loch durch eine Befestigungsschraube (16) gehalten wird.

7. Vorrichtung (1) nach dem vorhergehenden Anspruch, **dadurch gekennzeichnet, dass** jede der Haltespindeln (5a, 5b, 5c) auf der Querstange (13) durch Halteeinheiten (11a, 11b, 11c), die mit den Halteeinheiten (6a, 6b) identisch sind, die die Befestigung der Querspindeln (7a, 7b) ermöglichen, befestigt ist.

8. Fixateur (1) nach einem der vorhergehenden Ansprüche, **dadurch gekennzeichnet, dass** die Haltestange (4) von zwei Stangenelementen gebildet ist, nämlich einem unteren Stangenelement (4a) und einem oberen Stangenelement (4b), um eine Teleskopeinheit zu bilden, während sie Mittel zur Längeneinstellung (40) umfasst, die es ermöglichen, die Länge der Haltestange durch Längsverschiebung eines Stangenelements in Bezug zum anderen Element anzupassen.

## Claims

1. External holding device (1) intended for the repair and reduction of a fracture of the head of the humerus of a patient, by holding in place externally the two parts (2a, 2b) of the humerus (2), while it comprises a support rod (4) the top end of which comprises a top holding assembly (12) that is formed by a transverse rod (13) held at the top part of the support rod (4), **characterised in that** it comprises three holding pins (5a, 5b, 5c), one of the pins (5a) being fixed directly to the transverse rod (13), while the other two (5b, 5c) are fixed to a second transverse rod (13a) fixed to the transverse rod (13).

2. Device (1) according to the preceding claim, **characterised in that** the support rod (4) comprises two holding assemblies (6a, 6b), the latter each being intended to hold a transverse pin (7a, 7b) respectively intended to be impacted or screwed by their end into the body of the humerus (2b) in order to hold the support rod (4) so as to ensure fixing thereof without any possible movement.

3. Device (1) according to claim 2, **characterised in that** each of the holding assemblies (6a, 6b) is formed by a main member (8) and a secondary member (9).

4. Device (1) according to claim 3, **characterised in that** the main member (8) is a volume part advantageously having the split clevis form comprising a hole (80) for passage of the support rod (4) while a screw (10) provides the clamping of the clevis on the support rod.

5. Device (1) according to the preceding claim, **characterised in that** the secondary member (9) is associated with each main member (8), said secondary member (9) being formed, like the main member, by a split clevis fixed by clamping to the main member by means of the screw (10), while the secondary member (9) comprises a central hole (91) in which the transverse pin (7a, 7b) is engaged.

6. Device (1) according to the preceding claim, **characterised in that** the transverse rod (13) is held at the top part of the support rod (4) by means of a socket (14) pierced by at least one transverse hole (15) in which the transverse rod (13) is held advantageously in an inclined position so as to extend outwards and upwards, said transverse arm being held in the hole by means of a fixing screw (16).

7. Device (1) according to the preceding claim, **characterised in that** each of the holding pins (5a, 5b, 5c) is fixed to the transverse rod (13) by holding assemblies (11a, 11b, 11c) identical to the holding assemblies (6a, 6b) allowing fixing of the transverse pins (7a, 7b).

8. Device (1) according to any of the preceding claims, **characterised in that** the support rod (4) consists of two rod elements, namely a lower rod element (4a) and an upper rod element (4b), in order to form a telescopic assembly, while it comprises length-adjustment means (40) for adjusting the length of the support rod by longitudinal movement of one rod element with respect to the other element.
